(19) Europäisches Patentamt
European Patent Office
Office européen des brevets

(11) EP 1 043 012 A2

(12) **DEMANDE DE BREVET EUROPEEN**

(43) Date de publication:
**11.10.2000 Bulletin 2000/41**

(51) Int Cl.[7]: **A61K 7/13**

(21) Numéro de dépôt: **00400910.6**

(22) Date de dépôt: **03.04.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **07.04.1999 FR 9904338**

(71) Demandeur: **L'OREAL**
**75008 Paris (FR)**

(72) Inventeur: **Plos, Grégory**
**75015 Paris (FR)**

(74) Mandataire: **Casalonga, Axel**
**BUREAU D.A. CASALONGA - JOSSE**
**Morassistrasse 8**
**80469 München (DE)**

(54) **Procédé de teinture d'oxydation utilisant un cétose à titre d'agent réducteur et une laccase à titre d'agent oxydant**

(57) La présente invention a pour objet un procédé de teinture des fibres kératiniques et en particulier des fibres kératiniques humaines telles que les cheveux, caractérisé par le fait qu'il consiste

- à appliquer sur les fibres une composition de teinture (A) contenant, dans un milieu approprié pour la teinture, au moins un précurseur de colorant d'oxydation et/ou un ou plusieurs coupleurs et à titre d'agent réducteur au moins 0,1 à 15% en poids, par rapport au poids total de la composition(A) d'un cétose, et
- à révéler en présence d'air la couleur en milieu alcalin, neutre ou acide à l'aide d'une laccase, celle-ci étant incorporée dans la composition (A) ou dans une composition (B),

les compositions (A) et (B) étant mélangées immédiatement avant l'emploi ou appliquées l'une après l'autre sur les fibres kératiniques.

EP 1 043 012 A2

## Description

La présente invention concerne un procédé de teinture d'oxydation des fibres kératiniques, et en particulier des fibres kératiniques humaines telles que les cheveux, mettant en oeuvre des compositions comprenant, dans un milieu approprié pour la teinture, au moins un précurseur de colorant d'oxydation et/ou un ou plusieurs coupleurs, un cétose comme agent réducteur, et au moins une laccase à titre d'agent oxydant.

Il est connu de teindre les fibres kératiniques, et en particulier les cheveux, avec des compositions de teinture contenant des précurseurs de colorants d'oxydation, généralement appelés "bases d'oxydation", en particulier des ortho- ou para-phénylènediamines, des ortho- ou para-aminophénols et des bases hétérocycliques.

Les précurseurs de colorants d'oxydation sont des composés initialement peu ou pas colorés qui développent leur pouvoir tinctorial au sein du cheveu en présence d'un agent oxydant. L'agent oxydant utilisé est généralement le peroxyde d'hydrogène. La formation des composés colorés résulte, soit d'une condensation des "bases d'oxydation" sur elles-mêmes, soit d'une condensation des "bases d'oxydation" sur des composés modificateurs de coloration, ou "coupleurs", qui sont généralement présents dans les compositions tinctoriales utilisées en teinture d'oxydation et qui sont représentés plus particulièrement par des méta-phénylène-diamines, des méta-aminophénols et des méta-diphénols et certains composés hétérocycliques.

La variété des molécules mises en jeu, qui sont constituées d'une part par "les bases d'oxydation" et, d'autre part, par "les coupleurs", permet l'obtention d'une palette riche en coloris.

La coloration d'oxydation des fibres kératiniques peut également être réalisée à l'aide de systèmes oxydants différents du peroxyde d'hydrogène tel que des systèmes enzymatiques. Ainsi, il a déjà été proposé dans le brevet US 3,251,742, la demande de brevet FR-A-2 112 549, FR-A-2 694 018, EP-A-0 504 005, WO 95/07998, WO 95/33836, WO 95/33837, WO 96/00290, WO97/19998 et WO97/19999 de teindre les fibres kératiniques avec des compositions comprenant au moins un colorant d'oxydation en association avec des enzymes du type laccases, lesdites compositions étant mises en contact avec l'oxygène de l'air. En effet, il a été observé que l'eau oxygénée pouvait provoquer une dégradation de la fibre capillaire, et, en outre, une attaque partielle de la mélanine du cheveu, ce qui conduit à l'éclaircissement de la fibre.

Il a également été constaté que dans certains cas, les laccases permettaient d'obtenir des colorations d'oxydation satisfaisantes en utilisant uniquement des coupleurs, sans bases d'oxydation.

Ainsi, dans la présente invention, le terme colorant d'oxydation couvre les précurseurs de colorant d'oxydation et/ou les coupleurs.

Pour pouvoir conserver les colorants d'oxydation, c'est-à-dire les précurseurs de colorants d'oxydation et/ou les coupleurs, il est nécessaire de les associer à un réducteur.

Cependant, la demanderesse a constaté que ces réducteurs freinent généralement la montée des colorants sur les fibres, ce qui se traduit par des nuances moins lumineuses et des colorations moins puissantes.

Pour obtenir une chromaticité équivalente, il est alors nécessaire d'utiliser des quantités plus importantes de colorants.

De plus, de nombreux réducteurs utilisés jusqu'à présent, possèdent une action inhibitrice de l'activité de la laccase.

Après d'importantes recherches effectuées dans ce domaine, la demanderesse vient de découvrir que l'utilisation d'un cétose comme agent réducteur quand une laccase est utilisée comme agent oxydant permettait de résoudre les problèmes ci-dessus mentionnés.

En effet, il a été constaté que les cétoses n'inhibaient pas l'activité de la laccase; de plus, il a été constaté de façon surprenante que le mélange ainsi produit ne freinait pas la montée des colorants d'oxydation sur les cheveux.

Ces compositions donnent par ailleurs naissance à des nuances plus chromatiques (plus lumineuses) et à des colorations plus puissantes par rapport à des compositions équivalentes contenant des réducteurs et des agents oxydants habituels.

Les colorations obtenues présentent par ailleurs une bonne résistance à la transpiration, à la lumière et aux shampooings.

L'invention permet également de diminuer la quantité de matières actives colorantes utilisées dans les compositions de teinture par rapport aux techniques classiques et connues de l'art antérieur.

La présente invention a ainsi pour objet l'utilisation de 0,1 à 15 % en poids, par rapport au poids total de la composition(A) d'un cétose à titre d'agent réducteur et d'une laccase à titre d'agent oxydant pour la teinture oxydative.

Un autre objet de l'invention concerne un procédé de teinture des fibres kératiniques, et en particulier des fibres kératiniques humaines telles que les cheveux, consistant:

- à appliquer sur les fibres une composition de teinture (A) contenant, dans un milieu approprié pour la teinture, au moins un colorant d'oxydation et à titre d'agent réducteur au moins 0,1 à 15% en poids, par rapport au poids total de la composition(A) d'un cétose,

- à révéler en présence d'air la couleur en milieu alcalin, neutre ou acide à l'aide d'une laccase à titre d'agent oxydant, la laccase étant incorporée dans la composition (A), dans ce cas stockée à l'abri de l'air, ou dans une composition (B), les compositions (A) et (B) étant dans ce second cas mélangées immédiatement avant l'emploi ou appliquées l'une après l'autre sur les fibres kératiniques.

Le colorant d'oxydation est préférentiellement un précurseur de colorant d'oxydation avec éventuellement un ou plusieurs coupleurs.
Le colorant d'oxydation peut aussi être constitué d'un ou plusieurs coupleurs.
Dans un mode préféré de l'invention, le cétose est présent dans des proportions de 5 à 10% par rapport au poids total de la composition (A). Les cétoses selon la présente invention sont notamment des cétoses en $C_3$-$C_8$ et préférentiellement en $C_6$ (cétohexoses).
Comme exemples de cétoses, on peut notamment citer le xylulose, le ribulose, le fructose, le sédoheptulose, le tagatose, le sorbose ou le psicose.
Leurs isomères optiques (ou énantiomères), forme D ou L, peuvent être utilisés dans la présente invention, que ce soit sous forme pure (D ou L) ou sous forme de couple (D et L).
Le fructose (forme D et/ou L) est particulièrement préféré.
La ou les laccases utilisées dans le procédé conforme à l'invention peuvent notamment être choisies parmi les laccases d'origine végétale, d'origine animale, d'origine fongique (levures, moisissures, champignons) ou d'origine bactérienne, les organismes d'origine pouvant être mono ou pluricellulaires. Elles peuvent être obtenues par biotechnologie.
Parmi les laccases d'origine végétale utilisables selon l'invention, on peut citer les laccases produites par des végétaux effectuant la synthèse chlorophyllienne telles qu'indiquées dans la demande FR-A-2 694 018 comme celles que l'on retrouve dans les extraits des Anacardiacées tels que par exemple les extraits de Magnifera indica, Schinus molle ou Pleiogynium timoriense, dans les extraits des Podocarpacées, de Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.
Parmi les laccases d'origine fongique éventuellement obtenues par biotechnologie utilisables selon l'invention, on peut citer la ou les laccases issues de Polyporus versicolor, de Rhizoctonia praticola et de Rhus vernicifera comme indiquées dans les demandes FR-A-2 112 549 et EP-A-504005; celles décrites dans les demandes de brevet WO95/07988, W095/33836, WO95/33837, WO 96/00290, WO97/19998 et WO 97/19999, dont le contenu fait partie intégrante de la présente description comme par exemple celles issues de Scytalidium, de Polyporus pinsitus, de Myceliophtora thermophila, de Rhizoctonia solani, de Pyricularia orizae, ou leurs variantes. On peut aussi citer celles issues de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens et de leurs variantes.
On choisira plus préférentiellement les laccases d'origine fongique éventuellement obtenues par biotechnologie.
L'activité enzymatique des laccases de l'invention ayant la syringaldazine parmi leurs substrats peut être définie à partir de l'oxydation de la syringaldazine en condition aérobie. L'unité lacu correspond à la quantité d'enzyme catalysant la conversion de 1mmole de syringaldazine par minute à pH 5,5 à 30°C. L'unité u correspond à la quantité d'enzyme produisant un delta d'absorbance à 530 nm de 0,001 par minute en utilisant la syringaldazine comme substrat, à 30°C et à pH 6,5.
L'activité enzymatique des laccases de l'invention peut aussi être définie à partir de l'oxydation de la paraphénylènediamine. L'unité ulac correspond à la quantité d'enzyme produisant un delta d'absorbance à 496,5 nm de 0,001 par minute en utilisant la paraphénylènediamine comme substrat (64 mM) à 30°C et à pH 5.
Les quantités de laccase utilisées dans les compositions de l'invention varieront en fonction de la nature de la laccase choisie. De façon préférentielle, elles varieront de 0,5 à 3000 lacu, ou de 1000 à $6.10^7$ unités u; ou de 20 à $3.10^6$ unités ulac pour 100g de composition appliquée sur les cheveux.
Les colorants d'oxydation utilisables dans le cadre de la présente invention sont choisis parmi ceux classiquement connus en teinture d'oxydation.
On peut citer notamment les précurseurs de colorant d'oxydation suivants:

- les para-phénylènediamines de formule (I) suivante et les sels d'addition d'un acide de ces composés

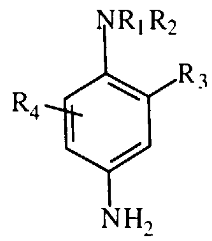

(I)

dans laquelle

$R_1$ représente un atome d'hydrogène, un radical alkyle en $C_{1-4}$, monohydroxyalkyle en $C_{1-4}$, polyhydroxyalkyle en $C_{2-4}$ ou 4'-aminophényle,
$R_2$ représente un atome d'hydrogène, un radical alkyle en $C_{1-4}$, monohydroxyalkyle en $C_{1-4}$ ou polyhydroxyalkyle en $C_{2-4}$,
$R_3$ représente un atome d'hydrogène, un atome d'halogène tel qu'un atome de chlore, un radical alkyle en $C_{1-4}$, sulfo, carboxy, monohydroxyalkyle en $C_{1-4}$ ou hydroxyalcoxy en $C_{1-4}$,
$R_4$ représente un atome d'hydrogène ou un radical alkyle en $C_{1-4}$.

Parmi les para-phénylènediamines de formule (I) ci-dessus, on peut citer en particulier la para-phénylènediamine, la para-toluylènediamine, la 2-chloro-para-phénylènediamine, la 2,3-diméthyl-para-phénylènediamine, la 2,6-diméthyl-para-phénylènediamine, la 2,6-diéthyl-para-phénylènediamine, la 2,5-diméthyl-para-phénylènediamine, la N, N-diméthyl-para-phénylènediamine, la N,N-diéthyl-para-phénylènediamine, la N,N-dipropyl-para-phénylènediamine, la 4-amino-N,N-diéthyl-3-méthylaniline, la N,N-bis(β-hydroxyéthyl)-para-phénylènediamine, la 4-amino-N,N-bis(β-hydroxyéthyl)-3-méthyl-aniline, la 4-amino-3-chloro-N,N-bis(β-hydroxyéthyl)-aniline, la 2-β-hydroxyéthyl-para-phénylènediamine, la 2-fluoro-para-phénylènediamine, la 2-isopropyl-para-phénylènediamine, la N-(β-hydroxypropyl)-para-phénylènediamine, la 2-hydroxyméthyl-para-phénylènediamine, la N,N-diméthyl-3-méthyl-para-phénylènediamine, la N-éthyl-N-(β-hydroxy-éthyl)-para-phénylènediamine, la N-(β,γ-dihydroxypropyl)-paraphénylènediamine, la N-(4'-aminophényl)-para-phénylènediamine, la N-phényl-para-phénylènediamine, la 2-β-hydroxyéthyloxy-para-phénylènediamine et les sels d'addition d'acide de ces composés.

Parmi les para-phénylènediamines de formule (I) ci-dessus, on préfère tout particulièrement la para-phénylènediamine, la para-toluylènediamine, la 2-isopropyl-para-phénylènediamine, la 2-β-hydroxy-éthyl-para-phénylèndiamine, la 2-β-hydroxyéthyloxy-para-phénylènediamine, la 2,6-diméthyl-para-phénylènediamine, la 2,6-diéthyl-para-phénylènediamine, la 2,3-diméthyl-para-phénylènediamine, la N,N-bis-(β-hydroxyéthyl)-para-phénylènediamine, la 2-chloro-para-phénylènediamine et les sels d'addition d'acide de ces composés.

- les bis-phénylalkylènediamines de formule (II) :

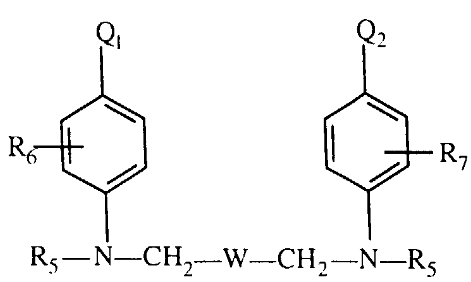

(II)

dans laquelle

$Q_1$ et $Q_2$, identiques ou différents, représentent un radical hydroxyle ou $NHR_8$ dans lequel $R_8$ représente un atome d'hydrogène ou un radical alkyle en $C_{1-4}$,
$R_5$ représente un atome d'hydrogène, un radical alkyle en $C_{1-4}$, monohydroxyalkyle en $C_{1-4}$, polyhydroxyalkyle en $C_{2-4}$ ou aminoalkyle en $C_{1-4}$ dont le groupe amino peut être substitué,
$R_6$ et $R_7$, identiques ou différents, représentent un atome d'hydrogène ou d'halogène ou un radical alkyle en $C_{1-4}$,

W représente un radical choisi dans le groupe formé par les radicaux suivants :
$-(CH_2)_n-$; $-(CH_2)_m-O-(CH_2)_m$; $-(CH_2)_m-CHOH-(CH_2)_m-$ et
$-(CH_2)_m-N(CH_3)-(CH_2)_m-$;

dans lesquels n est un nombre entier compris entre 0 et 8 inclusivement et m est un nombre entier compris entre 0 et 4 inclusivement, et les sels d'addition d'acide de tels composés.

Parmi les bis-phénylalkylènediamines de formule (II) ci-dessus, on peut citer en particulier le N,N'-bis(β-hydroxyéthyl)-N,N'-bis(4-aminophényl)-1,3-diamino-2-propanol, la N,N'-bis(β-hydroxyéthyl)-N,N'-bis(4-aminophényl)-éthylènediamine, la N,N'-bis(4-aminophényl)-tétraméthylènediamine, la N,N'-bis(β-hydroxyéthyl)-N,N'-bis(4-aminophényl)-tétraméthylènediamine, la N,N'-bis(4-méthylaminophényl)-tétraméthylènediamine, la N,N'-bis(β-hydroxyéthyl)-N,N'-bis(4-aminophényl)-tétraméthylènediamine, la N,N'-diéthyl-N,N'-bis(4-amino-3-méthylphényl)-éthylènediamine, et les sels d'addition d'acide de ces composés.

On recommande en particulier parmi ces bis-phénylalkylènediamines de formule (II) le N,N'-bis(β-hydroxyéthyl)-N,N'-bis(4'-aminophényl)-1,3-diamino-2-propanol ou l'un de ses sels d'addition d'un acide.

- les para-aminophénols répondant à la formule (III) :

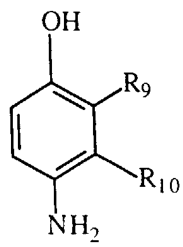

(III)

dans laquelle

$R_9$ représente un atome d'hydrogène, un radical alkyle en $C_{1-4}$, monohydroxyalkyle en $C_{1-4}$, (alcoxy en $C_{1-4}$)-(alkyle en $C_{1-4}$) ou aminoalkyle en $C_{1-4}$, ou hydroxy(alkyle en $C_{1-4}$)-aminoalkyle en $C_{1-4}$);
$R_{10}$, représente un atome d'hydrogène, un atome de fluor, un radical alkyle en $C_{1-4}$, monohydroxyalkyle en $C_{1-4}$, polyhydroxyalkyle en $C_{2-4}$, aminoalkyle en $C_{1-4}$, cyano(alkyle en $C_{1-4}$) ou (alcoxy en $C_{1-4}$)-(alkyle en $C_{1-4}$), et les sels d'addition d'acide de tels composés,

avec la réserve qu'au moins un des radicaux $R_9$ ou $R_{10}$ représente un atome d'hydrogène.

Parmi les para-aminophénols de formule (III) ci-dessus, on peut citer notamment le para-aminophénol, le 4-amino-3-méthylphénol, le 4-amino-3-fluorophénol, le 4-amino-3-hydroxyméthyl-phénol, le 4-amino-2-méthyl-phénol, le 4-amino-2-hydroxyméthylphénol, le 4-amino-2-méthoxyméthyl-phénol, le 4-amino-2-aminométhylphénol, le 4-amino-2-(β-hydroxyéthyl-aminométhyl)-phénol, et les sels d'addition d'acide de ces composés.

- les ortho-aminophénols utilisables à titre de bases d'oxydation dans le cadre de la présente invention sont notamment choisis parmi le 2-aminophénol, le 2-amino-1-hydroxy-5-méthylbenzène, le 2-amino-1-hydroxy-6-méthylbenzène, le 5-acétamido-2-aminophénol et les sels d'addition d'acide de ces composés;
- les bases hétérocycliques utilisables à titre de bases d'oxydation dans le cadre de la présente invention sont notamment choisies parmi les dérivés pyridiniques, les dérivés pyrimidiniques, les dérivés pyrazoliques et les sels d'addition d'acide de ces composés.

Parmi les dérivés pyridiniques, on peut plus particulièrement citer les composés décrits par exemple dans les brevets GB-1 026 978 et GB-1 153 196, comme la 2,5-diaminopyridine et les sels d'addition d'acide de tels composés.

Parmi les dérivés pyrimidiniques, on peut citer en particulier les composés décrits par exemple dans le brevet allemand DE-2 359 399 ou les brevets japonais JP-88-169 571, comme la 2,4,5,6-tétraaminopyrimidine, la 4-hydroxy-2,5,6,-triaminopyrimidine et les sels d'addition d'acide de tels composés.

Parmi les dérivés pyrazoliques, on peut plus particulièrement citer les composés décrits dans les brevets DE-3 843 892, DE-4 133 957 et demandes de brevet WO-94/08969 et WO-94/08970 comme le 4,5-diamino-1-méthylpyrazole, le 3,4-diamino-pyrazole, le 4,5-diamino-1-(4'-chlorobenzyl)-pyrazole et les sels d'addition d'acide de ces composés.

Selon l'invention, le ou les précurseurs de colorants d'oxydation représentent de préférence de 0,0005 à 12 % en poids du poids total de la composition (A) et encore mieux de 0,005 à 6 % en poids environ.

Les coupleurs utilisables dans le procédé de teinture selon l'invention sont ceux classiquement utilisés dans les compositions de teinture d'oxydation, c'est-à-dire des méta-phénylènediamines, des méta-aminophénols et des méta-diphénols (résorcinols), les dérivés mono- ou polyhydroxylés du naphtalène, le sésamol et ses dérivés et des composés hétérocycliques tels que, par exemple, les coupleurs indoliques, les coupleurs indoliniques, les coupleurs pyridiniques et les sels d'addition d'acide de tels composés.

Ces coupleurs peuvent notamment être choisis parmi le 2-méthyl-5-amino-phénol, le 5-N-(β-hydroxyéthyl)-amino-2-méthylphénol, le 3-aminophénol, le 1,3-dihydroxybenzène, le 1,3-dihydroxy-2-méthylbenzène, le 4-chloro-1,3-dihydroxybenzène, le 1-β-hydroxyéthoxy)-2,4-diaminobenzène, le 2-amino-4-(β-hydroxyéthylamino)-1-méthoxybenzène, le 1,3-diaminobenzène, le 1,3-bis(2,4-diaminophénoxy)-propane, le sésamol, l'α-naphtol, le 6-hydroxyindole, le 4-hydroxyindole, le 4-hydroxy-N-méthylindole, la 6-hydroxyindoline, la 2,6-dihydroxy-4-méthylpyridine, la 1-H-3-méthylpyrazol-5-one, la 1-phényl-3-méthyl-pyrazol-5-one et les sels d'addition d'acide de tels composés.

Lorsqu'ils sont présents, ces coupleurs représentent de préférence d'environ 0,0001 à 10 % en poids du poids total de la composition (A), et en particulier d'environ 0,005 à 5 % en poids.

D'une manière générale, les sels d'addition d'un acide des composés chromogènes, à savoir les bases d'oxydation et les coupleurs, sont choisis notamment parmi les chlorhydrates, les bromhydrates, les sulfates, les tartrates, les lactates et les acétates.

La composition (A) peut contenir, en plus des colorants d'oxydation définis ci-dessus, des colorants directs pour enrichir les nuances en reflets. Ces colorants directs peuvent notamment être choisis parmi les colorants nitrés, azoïques ou anthraquinoniques.

La composition (A) et/ou la composition (B) peuvent en outre contenir au moins un polymère substantif cationique ou amphotère tel que ceux définis dans EP-A-0 673 641, parmi lesquels on préfère avantageusement mettre en oeuvre :

- les polymères poly(ammonium quaternaire) préparés et décrits dans le brevet français 2 270 846, constitués de motifs récurrents répondant à la formule (IV) suivante :

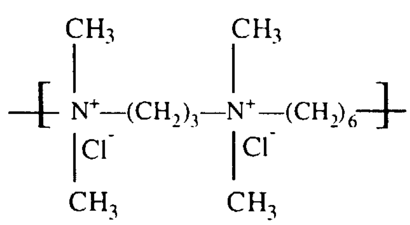

(IV)

et dont la masse molaire moyenne en poids, déterminée par chromatographie par perméation de gel, est comprise entre 9500 et 9900 ;

- les polymères poly(ammonium quaternaire) préparés et décrits dans le brevet français 2 270 846, constitués de motifs récurrents répondant à la formule (V) suivante :

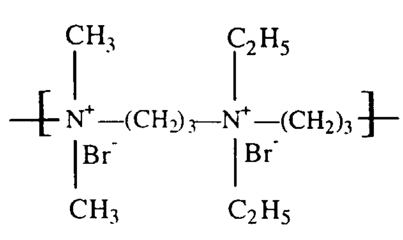

(V)

et dont la masse molaire moyenne en poids, déterminée par chromatographie par perméation de gel, est d'environ 1200.

Le milieu de la composition (A) approprié pour la teinture est de préférence un milieu aqueux constitué majoritairement d'eau et contenant, éventuellement, des solvants organiques acceptables sur le plan cosmétique, parmi lesquels figurent des alcools tels que l'alcool éthylique, l'alcool isopropylique, l'alcool benzylique et l'alcool phényléthylique; des glycols ou éthers de glycols tels que les éthers monométhylique, monoéthylique et monobutylique d'éthylèneglycol, le propylèneglycol ou ses éthers tels que l'éther monométhylique de propylèneglycol; le butylèneglycol; le dipropylèneglycol ainsi que les alkyléthers de diéthylèneglycol comme par exemple l'éther monométhylique ou monobutylique de diéthylèneglycol, en des concentrations comprises entre environ 0,5 et 20 % en poids, de préférence entre environ 2 et 10 % en poids, par rapport au poids total de la composition.

La composition (A) peut encore contenir une quantité efficace d'autres agents utilisés couramment dans le domaine de la teinture d'oxydation. Ces adjuvants sont par exemple des agents séquestrants, des agents de conditionnement du cheveu et en particulier des silicones, des agents conservateurs, des agents opacifiants etc., et éventuellement des agents tensio-actifs anioniques, non-ioniques, amphotères ou des mélanges de ceux-ci.

Bien entendu, l'homme de métier veillera à choisir le ou les éventuels composés complémentaires mentionnés ci-avant, d'une manière telle que les propriétés avantageuses attachées intrinsèquement à la composition tinctoriale selon l'invention ne soient pas, ou pratiquement pas, altérées par la ou les adjonctions envisagées.

Les valeurs du pH des compositions (A) et (B) peuvent notamment être choisies de manière à ce que la valeur du pH de la composition prête à l'emploi, résultant du mélange de la composition tinctoriale (A) et de la composition oxydante (B), soit généralement comprise entre 3 et 11, de préférence entre 4 et 9 et encore plus préférentiellement entre 6 et 8. Elles peuvent être ajustées au moyen d'agents acidifiants ou alcalinisants bien connus dans la technique de teinture d'oxydation des fibres kératiniques.

On peut citer parmi les agents alcalinisants, par exemple l'ammoniaque, les carbonates alcalins, les alcanolamines telles que les mono-, di- et triéthanolamines ainsi que leurs dérivés, les hydroxydes de sodium ou de potassium et les composés de formule (VI) suivante :

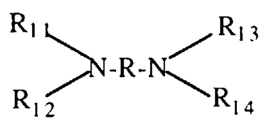

(VI)

dans laquelle R est un résidu propylène éventuellement substitué par un groupement hydroxyle ou un radical alkyle en $C_{1-4}$; $R_{11}$, $R_{12}$, $R_{13}$ et $R_{14}$, identiques ou différents, représentent un atome d'hydrogène ou un radical alkyle en $C_{1-4}$ ou hydroxyalkyle en $C_{1-4}$.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique ou des acides sulfoniques.

Un autre objet de la présente invention est une composition prête à l'emploi pour la teinture des fibres kératiniques contenant la laccase et le colorant d'oxydation et au moins 0,1 à 15% en poids, par rapport au poids total de la composition(A) d'un cétose ou susceptible d'être obtenue par mélange des compositions (A) et (B) ci-dessus définies.

L'invention a également pour objet un procédé de teinture des fibres kératiniques et en particulier des fibres kératiniques humaines telles que les cheveux mettant en oeuvre les compositions tinctoriales telles que définies précédemment.

Selon ce procédé, on applique sur les fibres au moins une composition tinctoriale prête à l'emploi telle que définie ci-avant pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

L'application de la composition tinctoriale prête à l'emploi peut avoir lieu notamment à une température comprise entre la température ambiante (20° C) et 60° C, et préférentiellement entre 35 et 50 °C.

Selon une forme de réalisation particulière de l'invention, le procédé comporte une étape préliminaire consistant à stocker sous forme séparée, d'une part, une composition (A) telle que définie ci-dessus et d'autre part, une composition (B) définie ci-avant puis à procéder à leur mélange au moment de l'emploi avant d'appliquer ce mélange sur les fibres kératiniques.

L'invention a également pour objet des dispositifs de teinture à plusieurs compartiments, ou "kits" de teinture comportant au moins deux compartiments, dont l'un contient une composition (A) contenant au moins un colorant d'oxydation et au moins 0,1 à 15% en poids, par rapport au poids total de la composition(A) d'un cétose et un autre contient une composition oxydante (B) contenant au moins une laccase. Ces dispositifs peuvent être équipés d'un moyen permettant de délivrer sur les cheveux le mélange souhaité, tel que les dispositifs décrits dans le brevet FR-2

586 913.

Il est bien entendu que la description qui précède n'a été donnée qu'à titre purement illustratif et non limitatif et que des variantes ou des modifications peuvent y être apportées dans le cadre de la présente invention.

Des exemples concrets illustrant l'invention vont maintenant être donnés sans pour autant présenter un caractère limitatif.

## EXEMPLES COMPARATIFS

On prépare les compositions tinctoriales suivantes (teneurs en grammes):

| EXEMPLE | 1 (*) | 2 (*) | 3 | 4 | 5 (*) |
|---|---|---|---|---|---|
| Paraphénylénediamine ($10^{-3}$ mole) | 0,108 g | 0,108 g | 0,108g | 0,108 g | 0,108 g |
| 1-méthyl-2 hydroxy-4 amino benzène ($10^{-3}$ mole) | 0,123 g | 0,123 g | 0,123 g | 0,123 g | 0,123 g |
| Fructose | - | - | 5 g | 10 g | - |
| Glucose | - | 5 g | - | - | - |
| Acide érythorbique | - | - | - | - | 0,3 g |
| Tampon phosphate commercialisé sous la dénomination Titrisol par la société Merck | pH 7 | pH 7 | pH 7 | pH 7 | pH 7 |
| Eau déminéralisée q.s.p. | (100-x) g | (100-x) g | (100-x) g | (100-x) g | (100-x) g |

(*) Exemples ne faisant pas partie de l'invention

On ajoute au moment de l'emploi x g d'une solution de laccase pour obtenir une composition tinctoriale finale ayant une concentration de laccase égale à $10^7$ unités u.

Puis, chacune des compositions tinctoriales obtenues a été appliquée sur des mèches de cheveux gris naturels à 90% de blancs, à raison de 5 g de composition par g de cheveux, pendant 30 minutes à 40° C. Les cheveux ont ensuite été rincés, lavés au shampooing, rincés à nouveau puis séchés.

Les cheveux teints avec les compositions 1*, 2*, 3, 4 et 5* présentaient la même nuance (pourpre rouge moyen).

Afin de déterminer de façon plus précise la montée de la coloration, la couleur des mèches a été évaluée avant et après la teinture dans le système MUNSELL, au moyen d'un colorimètre MINOLTA CM-2002®.

Selon la notation MUNSELL, une couleur est définie par l'expression H V/C dans laquelle les trois paramètres désignent respectivement la teinte ou Hue (H), l'intensité ou Value (V) et la pureté ou Chromaticité (C), la barre oblique de cette expression est simplement une convention et n'indique pas un ratio.

La différence entre la couleur de la mèche avant la teinture et la couleur de la mèche après la teinture exprime la puissance de la coloration et a été calculée en appliquant la formule de NICKERSON:

$$\Delta E = 0,4\ C0\Delta H + 6\Delta V + 3\Delta C$$

telle que décrite par exemple dans "Couleur, Industrie et Technique"; pages 14-17 ; vol. n° 5; 1978.

Dans cette formule, $\Delta E$ représente la différence de couleur entre deux mèches, $\Delta H$, $\Delta V$ et $\Delta C$ représentent la variation en valeur absolue des paramètres H, V et C et CO représente la pureté de la mèche par rapport à laquelle on désire évaluer la différence de couleur.

Plus la valeur de $\Delta E$ est élevée et plus la coloration est puissante.

Les résultats sont donnés dans le tableau ci-dessous:

| Composition | ΔE |
|---|---|
| 1 (*) | 30,94 |
| 2 (*) | 31,29 |
| 3 | 32,65 |
| 4 | 32.39 |

(suite)

| Composition | ΔE |
|---|---|
| 5 (*) | 5,75 |

Ces résultats montrent que la composition 2* ne faisant pas partie de l'invention et les compositions 3 et 4 conformes à l'invention conduisent à une coloration aussi puissante que celle de la composition 1* ne faisant pas partie de l'invention et qui ne contient pas d'agent réducteur. En revanche, la coloration obtenue avec la composition 5* utilisant l'acide érythorbique comme réducteur est faible. Ainsi, l'utilisation de cétose ne freine pas la montée de la coloration, et permet d'obtenir des colorations aussi puissantes que celles obtenues sans agent réducteur.
Les compositions tinctoriales 2*, 3 et 4 mentionnées ci-dessus ont également été conservées à une température ambiante de 22°C ± 2°C pendant 2 semaines.
Les mêmes colorations que celles précédemment décrites ont ensuite été réalisées.
Les résultats sont donnés dans le tableau suivant:

| Composition | ΔE |
|---|---|
| 2 (*) | 13,58 |
| 3 | 31,97 |
| 4 | 30,89 |

Ainsi, seule l'utilisation en tant que réducteur du fructose permet de réduire l'oxydation des précurseurs de coloration tout en ne modifiant pas dans le temps la montée de la coloration sur les fibres.

## Revendications

1. Utilisation d'au moins 0,1 à 15% en poids, par rapport au poids total de la composition d'un cétose à titre d'agent réducteur et d'une laccase à titre d'agent oxydant en teinture oxydative en présence d'au moins un colorant d'oxydation.

2. Procédé de teinture des fibres kératiniques et en particulier des fibres kératiniques humaines telles que les cheveux, caractérisé par le fait qu'il consiste:

   - à appliquer sur les fibres une composition de teinture (A) contenant, dans un milieu approprié pour la teinture, au moins un colorant d'oxydation et à titre d'agent réducteur au moins 0,1 à 15% en poids, par rapport au poids total de la composition(A) d'un cétose, et
   - à révéler en présence d'air la couleur en milieu alcalin, neutre ou acide à l'aide d'au moins une laccase incorporée dans la composition (A) ou dans une composition (B),

   les compositions (A) et (B) étant mélangées immédiatement avant l'emploi ou appliquées l'une après l'autre sur les fibres kératiniques.

3. Procédé selon la revendication 2, dans lequel le colorant d'oxydation de la composition de teinture (A) est un précurseur de colorant d'oxydation avec éventuellement un ou plusieurs coupleurs.

4. Procédé selon la revendication 2, dans lequel le colorant d'oxydation de la composition de teinture (A) est constitué d'un ou plusieurs coupleurs.

5. Procédé selon l'une quelconque des revendication 2 à 4, dans lequel le cétose est choisi parmi les cétohexoses, et est préférentiellement le fructose.

6. Procédé selon l'une quelconque des revendication 2 à 5, dans lequel la composition (A) contient préférentiellement de 5 à 10% en poids, par rapport au poids total de la composition (A), de cétose.

7. Procédé selon l'une quelconque des revendications 2 à 6, dans lequel la laccase est choisie parmi les laccases

d'origine végétale, d'origine animale, d'origine fongique, d'origine bactérienne, ou obtenues par biotechnologie.

**8.** Procédé selon l'une quelconque des revendications 2 à 7, dans lequel la laccase est choisie parmi celles produites par des végétaux effectuant la synthèse chlorophyllienne.

**9.** Procédé selon la revendication 8, dans lequel la laccase est choisie parmi celles extraites des Anacardiacées ou des Podocarpacées, des Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

**10.** Procédé selon la revendication 7, dans lequel la laccase est choisie parmi celles issues de Pyricularia orizae, de Polyporus versicolor, de Rhizoctonia praticola, de Rhus vernicifera, de Scytalidium, de Polyporus pinsitus, de Myceliophtora thermophila, de Rhizoctonia solani, de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, ainsi que leurs variantes.

**11.** Procédé selon l'une quelconque des revendications 2 à 10, dans lequel la laccase est présente dans des quantités allant de 0,5 à 3000 lacu, ou de 1000 à $6.10^7$, ou de 20 à $3.10^6$ unités ulac, pour 100g de composition prête à l'emploi.

**12.** Procédé selon l'une quelconque des revendications 3 et 5 à 12, dans lequel les précurseurs de colorants d'oxydation de la composition (A) sont choisis parmi les ortho- ou para-phénylènediamines, les bis-phénylalkylènediamines, les ortho- ou para-aminophénols, et les bases hétérocycliques ainsi que les sels d'addition d'un acide de ces composés.

**13.** Procédé selon la revendication 10, dans lequel les précurseurs de colorants d'oxydation sont présents à raison de 0,0005 à 12 % en poids par rapport au poids total de la composition (A).

**14.** Procédé selon l'une quelconque des revendications 3 à 13, dans lequel les coupleurs de la composition (A) sont choisis parmi les méta-phénylènediamines, les méta-aminophénols, les méta-diphénols, les coupleurs hétérocycliques et les sels d'addition d'un acide de ces composés.

**15.** Procédé selon la revendication 14, dans lequel les coupleurs sont présents à raison de 0,0001 à 10 % en poids par rapport au poids total de la composition (A).

**16.** Procédé selon les revendications 12 et 14, dans lequel les sels d'addition d'un acide des précurseurs de colorants d'oxydation et des coupleurs sont choisis parmi les chlorhydrates, les bromhydrates, les sulfates, les tartrates, les lactates et les acétates.

**17.** Procédé selon l'une quelconque des revendications 2 à 16, dans lequel la composition (A) contient en outre des colorants directs.

**18.** Procédé selon l'une quelconque des revendications 2 à 17, dans lequel la composition (A) et/ou (B) contient en outre au moins un polymère substantif cationique ou amphotère.

**19.** Procédé selon la revendication 18, dans lequel le polymère substantif est un polymère poly(ammonium quaternaire) constitué de motifs récurrents répondant à la formule (IV) suivante :

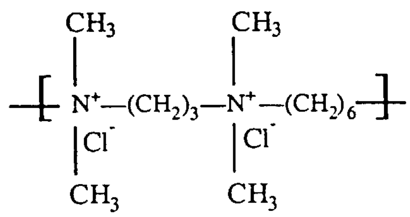

(IV)

**20.** Procédé selon la revendication 19, dans lequel le polymère substantif est un polymère poly(ammonium quaternaire) constitué de motifs récurrents répondant à la formule (V) suivante :

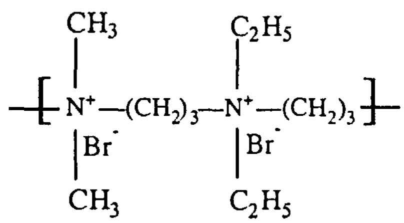

(V)

**21.** Procédé selon l'une quelconque des revendications 2 à 20, dans lequel la composition (A) contient, en outre, un ou plusieurs adjuvants choisis parmi les agents séquestrants, les agents de conditionnement du cheveu, notamment des silicones, les agents conservateurs, les agents opacifiants et les agents tensio-actifs anioniques, non-ioniques, amphotères ou leurs mélanges.

**22.** Procédé selon l'une quelconque des revendications 2 à 21, dans lequel la valeur du pH de la composition prête à l'emploi est comprise entre 3 et 11, de préférence entre 4 et 9, et encore plus préférentiellement entre 6 et 8.

**23.** Composition (A) telle que définie dans l'une quelconque des revendications 2 à 22.

**24.** Composition prête à l'emploi susceptible d'être obtenue par mélange des compositions (A) et (B) telles que définies dans l'une quelconque des revendications 2 à 22.

**25.** Procédé de teinture des fibres kératiniques et en particulier des fibres kératiniques humaines telles que les cheveux, caractérisé par le fait que l'on applique sur les fibres au moins une composition tinctoriale (A) selon la revendication 23 ou une composition tinctoriale prête à l'emploi selon la revendication 22, pendant un temps suffisant pour développer la coloration désirée.

**26.** Procédé caractérisé par le fait qu'il comporte une étape préliminaire consistant à stocker sous forme séparée, d'une part, la composition (A) selon la revendication 23 et, d'autre part, la composition (B) selon l'une quelconque des revendications 2 à 22, puis à procéder à leur mélange au moment de l'emploi avant d'appliquer ce mélange sur les fibres kératiniques.

**27.** Procédé selon la revendication 25 ou 26, dans lequel l'application de la composition tinctoriale prête à l'emploi est réalisée à une température comprise entre 20 et 60 °C et préférentiellement entre 35 et 50 °C.

**28.** Dispositif à plusieurs compartiments, ou "kit", pour la teinture des fibres kératiniques et en particulier des fibres kératiniques humaines telles que les cheveux, caractérisé par le fait qu'il comporte au moins deux compartiments dont l'un contient une composition (A) contenant au moins un colorant d'oxydation et au moins 0,1 à 15% en poids, par rapport au poids total de la composition(A) d'un cétose, et un autre compartiment contient une composition oxydante (B) contenant au moins une laccase.